# EUROPEAN PATENT SPECIFICATION

(11) **EP 0 671 625 B1**
(45) Date of publication and mention of the grant of the patent: **04.10.2000**
(21) Application number: 95103225.9
(22) Date of filing: 07.03.1995
(51) Int. Cl.: G01N 33/535, C12Q 1/42, C12Q 1/34, C12Q 1/68

(54) **A process for detecting biomolecules by means of an enzymatic amplification system**
Ein Verfahren zum Nachweis von Biomolekülen mit Hilfe eines enzymatischen Amplifizierungssystems
Procédé pour déterminer des biomolécules par un système d'amplification enzymatique

(30) Priority: 11.03.1994 IT TO940177
(43) Date of publication of application: 13.09.1995
(73) Proprietor: MINISTERO DELL' UNIVERSITA' E DELLA RICERCA SCIENTIFICA E TECNOLOGICA, 00196 Roma (IT)
(72) Inventor: Rigo, Adelio, I-30123 Venezia (IT); Bernacca, Giuliana,, I-54036 Marina Di Carrara (Massa Carrara (IT); Della Ciana, Leopoldo,, I-48022 Lugo (Ravenna) (IT)
(74) Representative: Rambelli, Paolo

(56) References cited:
- CHEMICAL ABSTRACTS, vol. 112, no. 7, 1990, February 12, Columbus, Ohio, USA J.E.FREW et al. "Measurement of alkaline phosphatase activity by electrochemical detection of phosphate esters. Application to amperometric enzyme immuno- assay" page 305, no. 50 972v; & J. Electroanal. Chem. Interfacial Electrochem. 1989, 266(2), 309-16.
- CHEMICAL ABSTRACTS, vol. 96, no. 11, 1982, March 15, Columbus, Ohio, USA J. KULYS et al.: "Determination of the activity of hydrolases" page 266, no. 81 894b; & SU-A-873 121
- CHEMICAL ABSTRACTS, vol. 95, no. 23, 1981, December 07, Columbus, Ohio, USA J. KULYS et al. "Substrate for determining the activity of alkaline phosphatase" page 302, no. 199 785z; & SU-A-853 536
- CHEMICAL ABSTRACTS, vol. 95, no. 19, 1981, November 09, Columbus, Ohio, USA V. RAZUMAS et al. "Study of alkaline phosphatase and beta-glucosidase in an electrochemical cell" page 332, no. 164 695t; & Proc. Jt. US/USSR Conf. Microb. Enzyme React. Proj. US/USSR Jt. Work. Group Prod. Subst. Microbiol. Means, 5th 1979 (Pub. 1980), 236-57.
- CHEMICAL ABSTRACTS, vol. 93, no. 9, 1980, September 01, Columbus, Ohio, USA V. RAZUMAS et al. "Kinetic amperometric determination of hydrolase activity" page 241, no. 90 745u; & Anal. Chim. Acta 1980, 117, 387-90.
- CHEMICAL ABSTRACTS, vol. 87, no. 19, 1977, November 07, Columbus, Ohio, USA B.C. AXCELL et al.: "Measurement of alkaline phosphatase levels in body fluids" page 242, no. 147 999n; & S. AFRICAN 75 06,003.

## Description

The present invention relates to a method for determining the presence or concentration of biomolecules, such as antibodies, antigens, nucleic acids or molecules having biological activity in general, in a sample by means of an enzymatic amplification system which uses an hydrolytic enzyme as a marker.

It is known that enzymes are used as probes for marking molecules of which it is wished to determine the concentration in a particular sample. Alkaline phosphatase is one of the markers most used for measurements in immune testing or in other areas of clinical diagnosis, for example as a marker for DNA probes. The known methods for detecting biomolecules marked with phosphatase include incubation of the marked biomolecule with a substrate which can be hydrolysed by the enzyme and detection of the product of the hydrolytic reaction by amperometry.

Various types of substrate have been reported for determining the activity of alkaline phosphatase, such as p-nitrophenyl phosphate and p-aminophenyl phosphate. Frew, J. E., et al., J. Electronal. Chem. Interfacial Electrochem, 1989, 266 (2), 309-316 discloses the use of p-aminophenyl phosphate in the electrochemical measurement of alkaline phosphatase activity in amperometric enzyme immunoassays. The substitution of the phosphate groups in the substrates by phenolic hydroxyl groups with the aid of the alkaline phosphatase varies the characteristics of the compound used as the substrate and enables the reaction products to be detected by amperometry. Thus, by measurement of the phenol formed in the reaction, the concentration or activity of the alkaline phosphatase can be determined. A large variety of substrates has been synthesised and tested in order to increase the sensitivity of the method, as illustrated by the following bibliographic references:
a) H.T.Tang, C.E.Lunte, H.B.Halsall, W.R.Heineman, Anal. Chim.Acta 214 (1988), 187-195;
b) J.Kulys, V. Razumas and Malinauskas J., Electroanal. Chem., 116, (1989), 11;
C) J. Kulys, V. Razumas and Malinauskas, Anal. Chim. Acta 117, (1980),387;
d) R.Q.Thompson, Anal-Chim.Acta,271, (1993), 223-339.

The problems which it is wished to solve by the present invention relate to the lowering of the detection threshold in the determination of a series of biomolecules, such as antibodies, antigens and nucleic acids, with the use of a marker enzyme with phosphatase hydrolytic activity. The term "enzyme with phosphatase hydrolytic activity" is intended to include all enzymes, including in particular, alkaline phosphatase itself, which are able to replace the phosphate group in a phosphoric ester by an hydroxyl group.

With this technical problem in mind, the primary object of the present invention is to provide reaction substrates for the biomolecules marked with the enzymes which enable the marked biomolecule, or molecules which interact therewith, to be detected at particularly low concentrations in comparison with those which can be measured with the substrates at present available, or which enable the required analysis times to be reduced by amplifying the signal generated by the action of the enzyme on the substrates themselves in that the lowering of the detection threshold enables the time needed for the amplification to be reduced.

In view of these objects, the subject of the invention is a method for determining the presence or concentration of a biomolecule in a test sample, wherein the biomolecule is marked with an enzyme having phosphatase hydrolytic activity or is included in a complex formed by two or more biomolecules, at least one of which is marked with said enzyme, and the biomolecule or complex is placed in contact with a substrate including a phenyl phosphate derivative which can be hydrolysed by the enzyme under conditions adapted to cause the hydrolysis, and in which the hydrolysis product is oxidised electrochemically in order to measure the oxidation current, characterised in that, as the substrate, there is used a medium which includes a compound having the general formula: 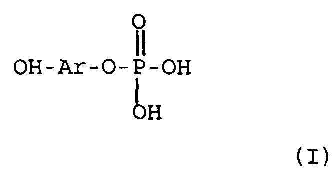 in which Ar is phenyl, unsubstituted or mono-, di- or trisubstituted with radicals selected independently of each other from C₁-C₄ alkyl, C₁-C₄ alkoxy, halogen, nitro (NO₂), sulphonic (SO₃H) and in which the hydroxyl group is in the para position relative to the phosphate group, and further characterised in that the electrochemical oxidation reaction is carried out in the presence of an enzymatic amplification system comprising glucose oxidase-glucose.

Preferred compounds are para-hydroxyphenyl phosphates, either unsubstituted or mono-substituted with methyl, ethyl, methoxy, ethoxy, chloro, NO₂, SO₃H.

The mono-phosphoric esters of diphenols of formula (I) are prepared from the corresponding diphenols after protection of one of the hydroxyl groups with a protector group, for example, with the use of a compound such as benzoyl chloride which can react with the hydroxyl group itself. The ester thus produced is placed in contact with a phosphorylating agent, for example phosphorus oxytrichloride, and then the phosphorylated derivative is treated with a base such as ammonia which can regenerate the phenol group protected initially.

In the presence of an enzyme with phosphatase hydrolytic activity, the compounds of formula (I) are hydrolysed, with the formation of the corresponding diphenols which have chemical and physical properties which differ from those of the enzymatic substrate and which may be determined electrochemically on an electrode polarised to a suitable potential, the diphenols formed in the reaction with the enzyme, being electrically active within a range of potentials (from -0.1050V to +1V with a silver-silver chloride electrode as the reference) which is considerably less than that of the oxidation-reduction of the starting phosphorylated compounds thus, contrary to the starting substrate, giving a measurable electric current.

In the method of the invention, the concentration of biomolecules or molecules having biological activity may be determined directly or indirectly by their marking with an enzyme as specified above, for example with the use of methods typically used for immune testing or for testing the hybridisation of nucleic acids, which enable specific sequences to be obtained by a chain reaction using polymerases.

One example of application of the method developed for immunoassay tests, which use antigens or antibodies specific for various biomolecules, is given below. In these tests, alkaline phosphatase, the marker molecule, is linked covalently to the antigen or antibody. After incubation of the antibody-antigen system, suitably immobilised and marked, formation of the specific link between the antibody and the antigen in the presence of the molecule to be analysed and removal of the molecules which have not reacted, the system is then placed in contact with an excess of the compound of formula (I) for a period of time which may vary from several seconds to several minutes. At the end of this second period of incubation, the diphenol formed by the catalytic action of the marker enzyme is determined electrochemically. More particularly, the working electrode, generally constituted by a metal surface (such as platinum, gold) or a carbonaceous material (such as graphite, carbon paste) is polarised to a working potential of between -0.15V and +1V relative to a saturate calomel electrode (reference electrode) and the oxidation current is measured.

In this stage a second enzymatic amplification system is added to the electrochemical cell, said amplification system comprising glucose oxidase-glucose which uses the quinone formed at the electrode as the oxidant of the glucose oxidase-glucose enzymatic system and enables the current to be increased even further and hence lowers the detection limit of the biomolecule to which the enzyme is conjugated. The increase in current obtained due to the diphenol generated in the incubation period and the subsequent amplification by the electrode-glucose-glucose oxidase system enables the quantity of the biomolecules marked with the enzyme, and hence that of the molecule to be analysed, to be calculated. Thus it is possible to determine particularly low quantities, even of the order of an attomole, of the molecule to be analysed.

### Example

To the system constituted by a specific antibody for an epitope of α-fetoprotein (molecule to be analysed) immobilised on the surface of a well, there was added a 0.1 ml sample of human plasma containing the molecule to be analysed at concentrations of 5 ng/ml and an excess of a second antibody specific for another epitope of α-fetoprotein. This second antibody was bonded covalently to the marker enzyme (conjugate), that is to the alkaline phosphatase. After a period of incubation of one hour, the excess conjugate was removed by washing. 0.1 ml of a 1 mM solution of p-hydroxyphenyl phosphate buffered to pH 9.5 with 20 mM borate were added to the well. After a period of incubation of one minute, 10µl aliquots of the incubated solution were added to an electrochemical cell which contained the second glucose-glucose oxidase amplification system constituted by 1 ml of a solution containing 0.1 M glucose and 0.5 mg of glucose oxidase buffered to pH 7 with 0.1 M phosphate. The increase in current obtained due to the diphenol generated in the second incubation period and the amplifying action of the electrode-glucose-glucose oxidase system was 0.95 ± 0.10 nA for each addition of 10µl of the incubation solution to the electrochemical measurement cell.

Also within the scope of the invention are diagnostic kits for immunological tests or amplification tests including a substrate including a compound of general formula (I), glucose, glucose oxidase, and an enzyme with phosphatase hydrolytic activity for marking a biomolecule.

## Claims

1. A method for determining the presence or concentration of a biomolecule in a test sample, wherein the biomolecule is marked with an enzyme having phosphatase hydrolytic activity or is included in a complex formed by two or more biomolecules, at least one of which is marked with said enzyme, the biomolecule or complex is placed in contact with a substrate including a phenyl phosphate derivative which can be hydrolysed by the enzyme under conditions adapted to cause the hydrolysis, and the hydrolysis product is electrochemically oxidised in order to measure the oxidation current, characterised in that, as the substrate, there is used a medium which includes a compound having the general formula: 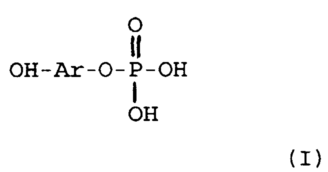 in which Ar is phenyl, unsubstituted or mono-, di- or trisubstituted with radicals selected independently of each other from C₁-C₄ alkyl, C₁-C₄ alkoxy, halogen, NO₂, SO₃H and in which the hydroxyl group is in the para position relative to the phosphate group, and further characterised in that the electrochemical oxidation reaction is carried out in the presence of an enzymatic amplification system comprising glucose oxidase-glucose.

2. A method according to Claim 1, in which Ar is unsubstituted phenyl or phenyl mono-substituted with a radical selected from the group consisting of methyl, ethyl, methoxy, ethoxy, chloro, NO₂, SO₃H.

3. A method according to Claim 1 or 2, in which the biomolecule is selected from the group consisting of antigens, antibodies and nucleic acids.

4. A method according to Claim 1 or 2, in which the biomolecule is an antibody-antigen complex.

5. A kit for use in a method according to any one of the preceding claims, comprising a compound of general formula (I) as defined in Claim 1, glucose, glucose oxidase and an enzyme with phosphatase hydrolytic activity for marking a biomolecule according to Claim 1.

## Patentansprüche

1. Verfahren zur Bestimmung der Anwesenheit oder Konzentration eines Biomoleküls in einer Testprobe, wobei das Biomolekül mit einem Enzym markiert ist, das eine Phosphatase hydrolytischer Aktivität aufweist oder in einem Komplex enthalten ist, der durch zwei oder mehr Biomoleküle gebildet ist, und zumindest eines von diesen mit dem erwähnten Enzym markiert ist, und das Biomolekül oder der Komplex in Kontakt mit einem Substrat gebracht wird, das ein Phenyl - Phosphat - Derivat einschließt, das durch das Enzym unter Bedingungen hydrolisiert werden kann, die zur Verursachung der Hydrolyse adaptiert sind und das Hydrolyseprodukt elektrochemisch oxidiert wird, um den Oxidationsstrom zu messen, **dadurch gekennzeichnet**, daß als Substrat ein Medium verwendet wird, das eine Verbindung mit der allgemeinen Formel enthält: 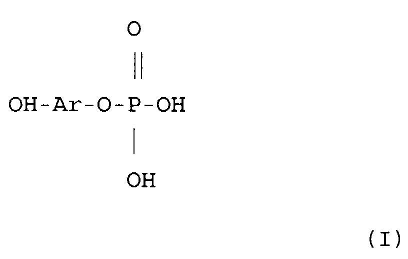 wobei Ar Phenyl, unsubstituiert oder mono-, di- oder trisubstituiert mit Radikalen ist, die unabhängig von einander aus C₁ - C₄ Alkyl, C₁ - C₄ Alkoxy, Halogenen, NO₂, SO₃H ausgewählt sind und wobei die Hydroxylgruppe in para-Position in Relation zur Phosphatgruppe steht und weiters dadurch gekennzeichnet ist, daß die elektrochemische Oxidationsreaktion in Gegenwart eines enzymatischen Amplifikationssystem durchgeführt wird, das Glukose Oxidase- Glukose enthält.

2. Verfahren nach Anspruch 1, in welchem Ar ein unsubstituiertes Phenyl oder ein mono - substituiertes Phenyl mit einem Radikal ist, das aus einer Gruppe, Methyl, Ethyl, Methoxy, Ethoxy, Chloro, NO2 und SO3H umfaßt, ausgewählt ist.

3. Verfahren nach Anspruch 1 oder 2, in welchem das Biomolekül aus einer Gruppe ausgewählt wird, die Antigene, Antikörper und Nukleinsäuren umfaßt.

4. Verfahren nach Anspruch 1 oder 2, in welchem das Biomolekül ein Antikörper - Antigen - Komplex ist.

5. Ein Kit zum Gebrauch in dem Verfahren nach einem der vorhergehenden Ansprüchen, der die Verbindung der allgemeinen Formel (I), wie in Anspruch 1 definiert, Glukose, Glukose- Oxidase und ein Enzym mit Phosphatase hydrolytischer Aktivität beinhaltet, um ein Biomolekül entsprechend dem Anspruch 1 zu markieren.

## Revendications

1. Procédé pour déterminer la présence ou concentration d'une biomolécule dans un échantillon d'essai, dans lequel la biomolécule est marquée par une enzyme ayant une activité hydrolytique de phosphatase ou est incorporée dans un complexe formé par deux biomolécules ou plus, dont l'une au moins est marquée par ladite enzyme, la biomolécule ou le complexe est mis(e) en contact avec un substrat comprenant un dérivé de phénylphosphate qui peut être hydrolysé par l'enzyme dans des conditions adaptées pour entraîner l'hydrolyse, et le produit d'hydrolyse est oxydé par voie électrochimique afin de mesurer le courant d'oxydation, caractérisé en ce que, en tant que substrat, on utilise un support qui comprend un composé répondant à la formule générale : 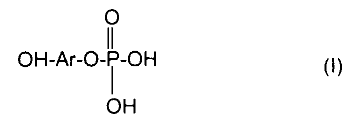 dans laquelle Ar représente un groupe phényle, non substitué ou mono-, di ou tri-substitué par des radicaux choisis indépendamment les uns des autres parmi un groupe alkyle en C₁ à C₄, alcoxy en C₁ à C₄, un atome d'halogène, NO₂, SO₃H et dans laquelle le groupe hydroxyle est en position para par rapport au groupe phosphate et caractérisé, en outre, en ce que la réaction d'oxydation électrochimique est réalisée en présence d'un système d'amplification enzymatique comprenant un composé glucose oxydase-glucose.

2. Procédé selon la revendication 1, dans lequel Ar représente un groupe phényle non substitué ou phényle monosubstitué par un radical choisi dans le groupe constitué par les groupes méthyle, éthyle, méthoxy, éthoxy, chloro, NO₂, SO₃H.

3. Procédé selon la revendication 1 ou 2, dans lequel la biomolécule est choisie dans le groupe constitué par les antigènes, les anticorps et les acides nucléiques.

4. Procédé selon la revendication 1 ou 2, dans lequel la biomolécule est un complexe anticorps-antigène.

5. Kit à utiliser dans un procédé selon l'une quelconque des revendications précédentes, comprenant un composé de formule générale (I) telle que définie dans la revendication 1, du glucose, de la glucose oxydase et une enzyme ayant une activité hydrolytique de phosphatase pour marquer une biomolécule selon la revendication 1.
